Europäisches Patentamt

European Patent Office

Office européen des brevets

(11) Publication number: 0 352 026
A2

# EUROPEAN PATENT APPLICATION

(21) Application number: 89307140.7

(22) Date of filing: 14.07.89

(51) Int. Cl.4: G01N 1/30 , G02B 21/34 , G01N 33/53

(30) Priority: 19.07.88 US 221339

(43) Date of publication of application:
24.01.90 Bulletin 90/04

(84) Designated Contracting States:
DE FR GB

(71) Applicant: Bonderman, Ruby Pauline
8340 East 126th Street
Noblesville Indiana 46060(US)

(72) Inventor: Bonderman, Ruby Pauline
8340 East 126th Street
Noblesville Indiana 46060(US)

(74) Representative: Bannerman, David Gardner et
al
Withers & Rogers 4 Dyer's Buildings Holborn
London, EC1N 2JT(GB)

(54) A glass slide useful as a control of standard by having several areas of differing levels or types of analytes, and devices and methods for preparing the same.

(57) This invention relates to an assay device useful as a control or standard for analyzing a biomaterial comprising:
a glass slide having a surface
indicia which divide said surface into several separate identifiable areas; and
biomaterial placed into said several areas with only one type in a known amount in each of said several areas, differing from the type or amount in each other of said several areas, whereby procedures and equipment can be checked for proper function.

EP 0 352 026 A2

# A GLASS SLIDE USEFUL AS A CONTROL OR STANDARD BY HAVING SEVERAL AREAS OF DIFFERING LEVELS OR TYPES OF ANALYTES, AND DEVICES AND METHODS FOR PREPARING THE SAME

## Background of the Invention

This invention relates generally to the field of biology, and more particularly to assay devices which are useful as controls or standards in quantitative and qualitative staining procedures relating to cytochemistry and/or histochemistry, and methods and devices for preparing the same.

It is often necessary in medical diagnosis or in scientific study to determine aspects of structure, function, and/or reactivity of cell, cell component, or tissue samples. To do this, many sophisticated staining procedures have been developed which capitalize on the ability of a stain to bind to a material to be determined in a sample. Both general and specific staining techniques have been developed, and each provides advantages in qualitative and quantitative analysis of samples.

For example, the field of immunostaining capitalizes on the ability of antibodies to bind with specificity to antigens. When it is necessary to determine whether a cell, cell component, or tissue sample contains a particular antigen, a stain comprising labeled antibodies which specifically bind to that antigen can be applied to the cell or tissue sample which may thereafter be checked for staining. Typically, the label of the antibody involves an attached fluorescent, colorimetric, or radioactive marker. If procedures and equipment are functioning properly, positive staining should indicate the presence of the antigen in the sample, and a negative staining result should indicate that the sample lacks the antigen of interest altogether, or perhaps contains a low level of the antigen insufficient for determination with the staining procedure used.

A major problem in all staining procedures is the interpretation of a negative result. A negative result can occur from improper preparation or dilution of reagents, sample preparative procedures, or mistakes in technique. Negative results are particularly troublesome in the diagnostic laboratory where material may be scarce and experience with a particular stain may be limited. In the field of immunochemistry, many experienced laboratories maintain a collection of positive tissue blocks for common antigens; however, these samples have the disadvantage that only a single concentration of antigen is available, and there is no effective way to assess whether a sample contains abundant or sparse antigen-binding sites, or the range of analyte concentration for which a particular staining procedure is effective.

The prior art in the field of staining includes many examples of sample staining-type assay devices and methods for their preparation, but there is a distinct omission in the prior art in the development of suitable, accurate and convenient controls or standards by which these assay devices and the processes surrounding their use and preparation may be monitored. These processes and devices most often involve the use of microtiter-type slides or tubes into which a staining agent and/or a sample is introduced. This type of arrangement, while effective for the processing of samples to be determined, would be inconvenient and wasteful if used to develop a control or standard device.

For example, U.S. Patent No. 4,713,352 issued to Bender et al. discloses new monoclonal antibodies which may be labeled with a fluorescent or radioactive agent, and can be used in a method to determine histiogenesis as well as prognosis of renal carcinoma subsets. Bender discloses that the new monoclonal antibodies may be used in a panel which may consist of monoclonal antibodies $S_4$(HB 8541), $S_{23}$(HB 8540), $F_{23}$(HB 8231), $S_{27}$(HB 8428) and $F_{31}$(HB 8548). Regarding the "panel", Bender discloses procedures whereby the antibodies are added to the wells of microtiter-type supports.

U.S. Patent No. 4,514,508 issued to Hirschfeld discloses the preparation of a microtiter plate having a qualitative and/or quantitative spectrum of antibodies, or spectrum of antigens or haptens, bound in each sample well. As described in the Hirschfeld patent, the microtiter plate is made of PVC, and although the patent recites that other supports might be used, it fails to disclose any others. The Hirschfeld patent also discloses that if multiple antibodies or portions thereof are used each different antibody is placed in a different well of the microtiter plate. This microtiter plate, as disclosed in the patent, is then used in methods or diagnostic packs which employ the principle of the recognition by a detection compound of antigen-antibody complexes which are formed when a test sample is added to the wells.

U.S. Patent No. 4,591,570 issued to Tse-Wen Chang discloses an assay device which essentially comprises a pattern or array of minute antibody-coated spots on the surface of a support such as a glass or plastic coverslip. As disclosed in the patent, the spots serve as tiny, specific immunoadsorbents of cells, and the expression of particular surface antigen by cells may be detected by determining to which antibody spot the cells bind. The patent describes that the antibody spots should be closely spaced and states that a 10 spot by 10 spot matrix should occupy about 1 cm² or less of the surface of the support, and that each spot, or each of certain sets of spots, is made up of antibodies of single distinct specificity.

U.S. Patent No. 4,357,142 issued to Schall, Jr. et al. discloses the application of adhesive films of organic-solvent based polymers to insoluble support materials for the purposes of adsorbing or covalently bonding biomaterials to the supports. As disclosed by the Schall patent, the solid support means may be comprised of glass, ceramics, metals, plastics, wood, etc., or any solid support material capable of being coated by the polymeric film means. However, the examples given describe only the use of glass tubes, a porcelain plate having wells, and steel spatulas as supports.

U.S. Patent No. 4,640,897 to Leynadier et al. relates to a diagnosis reagent for parasitoses and allergies, notably worm parasitoses which cause an increase in the circulating specific immunoglobulins, such as IgE. The reagent is characterized in that it comprises a suspension of specific IgE-carrying basophil granulocytes. Processes and kits are disclosed which involve depositing the reagent in a dry or wet state in the various wells of a diagnosis read-out slide or the like. It is described that the antigen present in the wells may be introduced at various concentrations, and an example with three concentrations is given.

As is evident from the above discussion, the histochemical and cytochemical staining and assaying arts have developed many sample assay devices and methods which employ various analytes combined with modified supports. Contemplating large scale production, distribution, and use, these prior art assay devices would each suffer from one or more of many disadvantages including the use of wastefully excessive amounts of reagents, difficulty in handling and viewing with accuracy test results, difficulty in packaging in that the supports are irregularly shaped and ampules of reagents must be included, and the lack of sufficiently varied testing sites to enable highly quantitative and qualitative analysis of samples and/or staining procedures, reagents, and equipment. Thus, it is evident that there is lacking in these arts the development of a convenient, accurate control or standard device which is prepared from common, inexpensive materials, and which provides both qualitative and highly quantitative analysis of staining or assay procedures, reagents, and equipment for proper function. Also lacking in the cytochemical and histochemical arts is the development of efficient and effective devices and methods for large-scale preparation of control or standard devices which provides substantial savings of time, labor, and expense. Such devices and methods have been long needed in these arts, particularly in light of the development of numerous families of standard stains and procedures. The applicants' invention addresses this need.

## Summary of the Invention

One aspect of the present invention involves a novel assay device useful as a control or a standard for analyzing a biomaterial. Such an assay device includes a glass slide having a surface divided by indicia into several separate identifiable areas. Biomaterial is placed into the several areas of the slide with only one type in a known amount in each of the several areas, differing from the type or amount in each of the other several areas.

Another aspect of the applicants' invention involves an assay device useful as a control or standard for analyzing an antibody, antigen, enzyme, or hormone receptor. Such an assay device includes a glass slide having a surface having several separate identifiable areas. An analyte selected from the group consisting of antibody, antigen, enzyme, and hormone receptor is placed into the several areas with only one uniform concentration of the analyte in each of the several areas. However, each of the several areas has differing concentrations of the analyte from the other of the several areas.

Another aspect of the present invention involves a multi-tipped applicator device for placing uniform samples of biomaterial into several separate identifiable areas of a glass slide. The multi-tipped applicator device includes a charging station having several separate wells for holding sample, said charging station being operably associated with a first vertical guide means, a base piece for supporting a glass slide and operably associated with a second vertical guide means, a sample delivery piece for supporting several applicator tips wherein each of said tips is independently vertically displaceable, said sample delivery piece being displaceable along said first vertical guide means toward and away from said charging station to cause said applicator tips to temporarily contact said sample solutions in said wells of said charging station thus charging said applicator tips with sample by surface tension, said sample delivery piece also being vertically displaceable along said second vertical guide means toward and away from said glass slide supported by said base piece to cause said applicator tips to contact said glass slide and thus deposit said sample on said glass slide, and a top piece displaceable along said first and second vertical guide means and designed to facilitate displacement of said sample delivery piece along said first and second vertical guide means.

Another aspect of the applicants' invention involves a method of preparing an assay device useful as a control or standard for analyzing a biomaterial. This aspect of the invention includes the steps of selecting a glass slide, and then using a multi-tipped applicator device to place biomaterial into several separate identifiable areas of the glass slide, with each applicator tip of the multi-tipped applicator device placing one type of biomaterial in a known amount into one of the several areas, differing from the type or amount of the biomaterial in each of the other several areas.

Another aspect of the invention involves a method of preparing an assay device useful as a control or standard for analyzing a biomaterial. This aspect of the applicants' invention is highlighted by the steps of selecting a glass slide, coating at least a portion of one of its surfaces with gelatin, and placing differing types or amounts of biomaterial in known amount into each one of the several separate identifiable areas of the glass slide on the surface coated with gelatin so that each of the several areas has only one type or amount of the biomaterial.

Still another aspect of the present invention involves a method of preparing an assay device useful as a control or standard for analyzing a biomaterial. This method is highlighted by the steps of providing a glass slide, and placing differing types or amounts of biomaterial in known amount into first and second areas of the glass slide. Each of the first and second areas receives only one type or amount of biomaterial, and the first and second areas intersect forming a third area which contains a combination of said biomaterial placed in the first and second areas.

Accordingly, one object of the present invention is to provide a standard or control for analyzing a biomaterial which is convenient, accurate, and amenable to large-scale production, distribution, and use, and which enables highly qualitative and quantitative determinations.

Another object of the present invention is to provide a convenient and accurate standard or control which permits evaluation of the effectiveness of a cytochemical or histochemical staining procedure.

Another object of the present invention is to provide improved multi-tipped applicator devices and methods for preparing assay devices which result in substantial savings of time, labor, and expense.

Another object of the present invention is to provide an improved method for preparing a mechanically stable, convenient assay device which involves coating a slide with gelatin prior to applying a biomaterial to several separate identifiable areas of the slide.

## Brief Description of the Drawings

FIG. 1 is a perspective view of an assay device marked numerically and with indicia.

FIG. 2 is a perspective view illustrating the base Piece of applicants' preferred multi-tipped applicator device.

FIG. 3 is a perspective view of the applicant's preferred applicator tip.

FIG. 4 is a perspective view of a sample delivery piece of the applicants' preferred multi-tipped applicator device supporting two applicator tips.

FIG. 5 is a perspective view of a sample delivery piece of the applicants' preferred multi-tipped applicator device supporting six applicator tips.

FIG. 6 is a perspecive view of the top piece, sample delivery piece, and base piece of the applicants' preferred multi-tipped applicator device.

FIG. 7 is a right-end view of FIG. 5 with the upwardly extending lips of the base piece broken away.

FIG. 8 is a perspective view of the charging station of the applicants' preferred multi-tipped applicator device.

FIG. 9 is a perspective view of a sample delivery piece of the applicants' multi-tipped applicator device capable of placing biomaterial in an arrangement of three rows of two abreast, as is illustrated in FIG. 1.

FIG. 10 is a cross-sectional view of a gelatin coated assay device.

FIG. 11 is a top view of a portion of a surface of a glass slide prepared using the applicants' Cross Pattern Method.

## Description of the Preferred Embodiment

For the purposes of promoting an understanding of the principles of the invention, reference will now be made to several aspects of the applicants work and specific language will be used to describe the same. It will nevertheless be understood that no limitation of the scope of the invention is thereby intended, such alterations and further modifications, and such further applications of the principles of the invention therein being contemplated as would normally occur to one skilled in the art to which the invention relates.

**Assay Device Marked With Indicia**

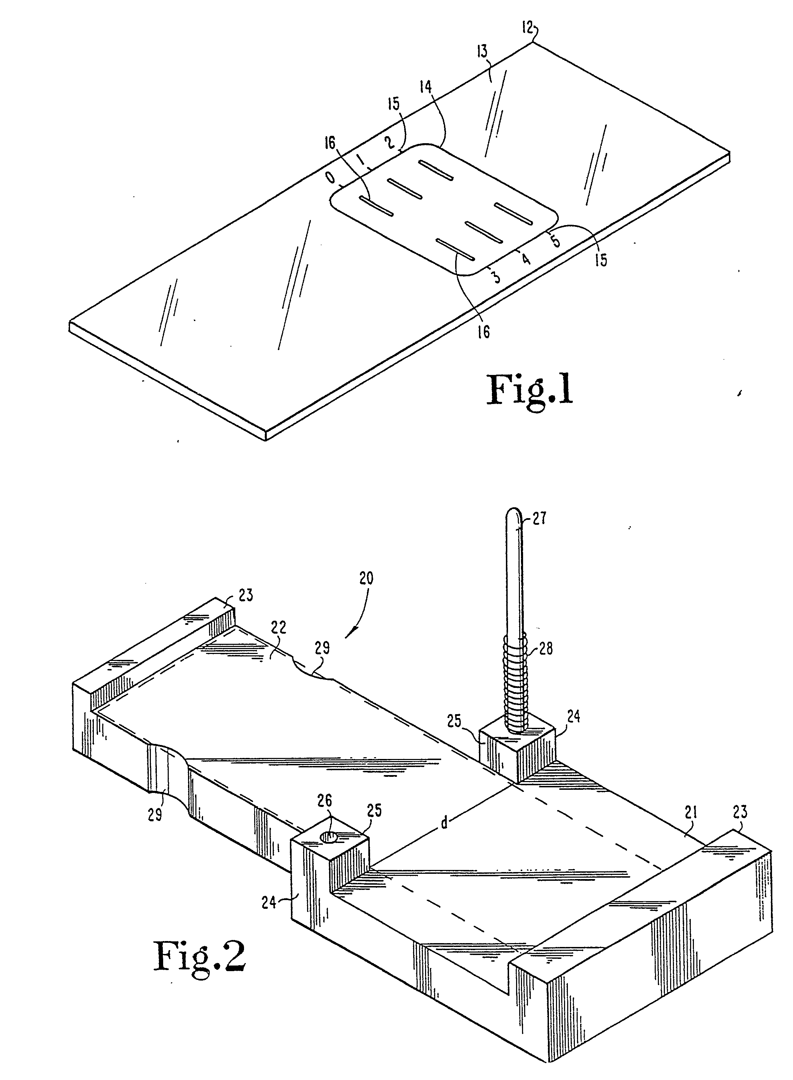

Referring now to FIG. 1, in accordance with the above discussion, one preferred aspect of the applicants' invention involves a novel assay device 11 useful as a control or a standard for analyzing a biomaterial. As used herein, "biomaterial" means any material which demonstrates biological activity. Such an assay device 11 includes a glass slide 12 having a surface 13 provided with a sample boundary marking 14 and divided by indicia 15 into several separate identifiable areas. Biomaterial 16 is placed into the several areas of the slide 12 with only one type in a known amount in each of the several areas, differing from the type or amount in each of the other several areas. In such a manner, the device may be used to check procedures and equipment for proper function.

In one mode of practicing the above preferred aspect, the glass slide may be obtained having individually numbered areas with numbers (0-5) which correspond with the indicia 15 and can be viewed microscopically. Such glass slides are available from the Erie Scientific Company (Portsmouth, New Hampshire) under the name Superfrost Square Well With Numbers. It is also contemplated that a proper procedure could be adopted to mark the glass slide with indicia and/or numbers after it is obtained, with one simple way being applying marked transparent tape sections to the glass slide. The indicia may comprise any visible marking on the slide, including simply lines as in FIG. 1, or could also include a grid-type marking on the slide. Additionally, any representative scheme of symbols, including alphabetical letters and the like, could be used in the place of the numerals.

The glass slide is preferably pre-treated and coated with a layer of gelatin, as described in the description and Examples below. However, it is contemplated that non-treated and non-gelatin-coated slides may also be suitable for use in the applicants' invention.

Any suitable biomaterial may be used in the applicants' invention. For example, the biomaterial may comprise cells, such as cells for PAP testing, parts of cells, viruses, antigens, antibodies, enzymes, hormone receptors, and nucleic acids such as RNA and DNA. Antigens including Immunoglobulin (Ig)G, IgM, IgK, gammaglobulin, IgA, IgE, Kappa chains, the intermediate filaments, prostate specific antigen, S-100 and Carcinoembryonic antigen could be used in the invention. Hormone receptors such as insulin, glucagon, and estrogen, enzymes such as cholinesterase, both single stranded and double stranded DNA and RNA, and PAP cells numbers 0-4 could be used in this preferred aspect as well. Other biomaterials may be suitable for use in the applicants' invention, and no limitation is intended by listing those above.

To date, the applicants have preferably used Micro-Tips available from Helena Laboratories (catalog number 4087) to apply the biomaterials to the slide. These tips each effectively deliver about 0.25 $\mu$l of sample to the slide per application, and deposit the material in a generally rectangular area and uniformly across such area. A more detailed description of the Micro-Tips is given below. When it is desirable to deliver larger quantities of sample to the slide, Macro-Tips, which are also available from Helena Laboratories (catalog number 4039) and which deliver approximately 0.5$\mu$l of sample may also be used. More specifically, the tips have been contacted with a solution of the biomaterial and then used to place the biomaterial on the slides.

To date, it has been preferred by the applicants to treat the biomaterials, once on the slide, with a suitable fixative. In this regard, formalin vapor has shown to be a preferred fixative for fixing antigens, while alcohol has been a preferred fixative for DNA, although 10% buffered formalin vapor is usable. To fix cholinesterase, the more preferred method has been to dehydrate the material with graded alcohol, with less preferred methods being immersion in a 10% buffered formalin solution, or treating with 10% buffered formalin vapor. When formalin vapor has been used as a fixative, applicants have preferred to fix for about 10 to 30 minutes, and most preferably about 20 minutes. Other fixatives and the biomaterials for which they may be used are known by those of ordinary skill in the art, and thus are included in the applicants' invention. After the fixation step, the applicants have preferably dried the slides at elevated temperatures, most preferably at about 37° C.

In one mode of practicing this preferred aspect of the invention, the biomaterial is placed in differing concentrations in each of the several separate identifiable areas of the glass slide. For example, the applicants have prepared gelatin-coated slides having samples from serial dilutions of IgG placed in each of 9 separate identifiable areas on the slide, with each area receiving a different concentration. An assay device was thus formed useful as a control or standard for quantitative and/or qualitative testing for the presence of the antigen IgG in an unknown, or for checking IgG assay procedures, reagents, or equipment for proper function. In a more preferred mode, the glass slide includes a separate identifiable area lacking the biomaterial of interest, to be used as a negative control. For example, five of the nine above-described IgG dilutions were placed in five numerically marked separate identifiable areas of a gelatin-coated glass slide. A sixth numerically marked separate identifiable area was spotted with a 1% Bovine Serum Albumin solution to form a negative control.

In another preferred mode of practicing this aspect of the invention, differing types of biomaterial are placed in the several areas of the glass slide. For example, slides having single stranded DNA at three numerically marked separate identifiable areas of the slide, and double stranded DNA at three other such areas have been prepared. Such an arrangement is valuable in qualitative analysis of unknowns, and it is understood that other differing types of biomaterials, including but not limited to PAP cells 0-4, antibodies, enzymes, nucleic acids, antigens, and hormone receptors, may be used in this mode.

### Control or Standard for an Antigen, Antibody, Enzyme, or Hormone Receptor

Another preferred aspect of the applicants' invention involves an assay device useful as a control or standard for analyzing an antibody, antigen, enzymes, or hormone receptor. Such an assay device includes a glass slide having a substantially planar surface having several separate identifiable areas. An analyte selected from the group consisting of antibody, antigen, enzyme, and hormone receptor is placed into the several areas. Each of the several areas has only one concentration of the analyte, uniform within each individual area. However, each of the several areas has differing concentrations of the analyte from the other of the several areas. In this manner, procedures and equipment can be checked for proper function in assaying the selected analyte, and a control can be formed to which unknowns can be compared.

Many antigens, antibodies, enzymes, and hormone receptors, are known in the art and could be used, including, for example, those described above. The differing concentrations used in this aspect would vary, of course, depending upon what levels of concentration for which one desires to assay an unknown or staining procedure. In a more preferred form, the glass slide includes at least 6 separate identifiable areas, with at least 5 differing concentrations of the antibody, antigen, enzyme, or hormone receptor of interest, and 1 area lacking the same to be used as a negative control. The areas may be identified by an suitable means, including using a system wherein slides are prepared which include the areas in a consistent pattern from one slide to the next, and that pattern is separately recorded (e.g. written on a separate document). Also, indicia and/or numerical markings can be used to divide the slide into the several separate identifiable areas. Preferably, the several areas from a generaly rectangular array, and when 6 separate identifiable areas are included, this array may consist of three rows each consisting of two separate identifiable areas.

As in the aspects discussed above, it has been preferred to date to pre-treat the glass slide and coat it with a layer of gelatin, and to fix the analytes with a suitable fixative after their application to the slide, as is set forth in the description and Examples below. Additionally, it has been preferred to date to use the above-described Micro-Tips and Macro-Tips available from Helena Laboratories to deliver the biomaterials to the slides.

### Multi-Tipped Applicator Device

Another preferred aspect of the applicants' invention involves a multi-tipped applicator device which is useful for preparing assay devices which are useful as controls or standards. Referring now to FIG. 2, shown is the base piece 20 of a preferred multi-tipped applicator device. The base piece 20 has a wide surface portion 21 and a narrow surface portion 22, with raised lip elements 23 at each end. Near the center of the base piece 20 and at the end of the wide surface portion 21 proximal to the narrow surface portion 22 are raised support blocks 24. The blocks 24 are located and are of a size such that the distance d between the inner surfaces 25 of the blocks 24 is substantially equal to the width nw of the narrow surface portion 22. The support blocks 24 are each fashioned with a hole 26, and the holes 26 are of a size such that guide bars 27 fit snugly into them. Over each guide bar is placed a soft spring 28. So designed, the base piece 20 may effectively support a glass slide (contours indicated with dotted lines) for treatment with the applicants' preferred multi-tipped applicator device. In a more preferred embodiment, base piece 20 has curved indentions 29 located at the edges of its more narrow end to facilitate the placement and removal of glass slides on and from the base piece 20.

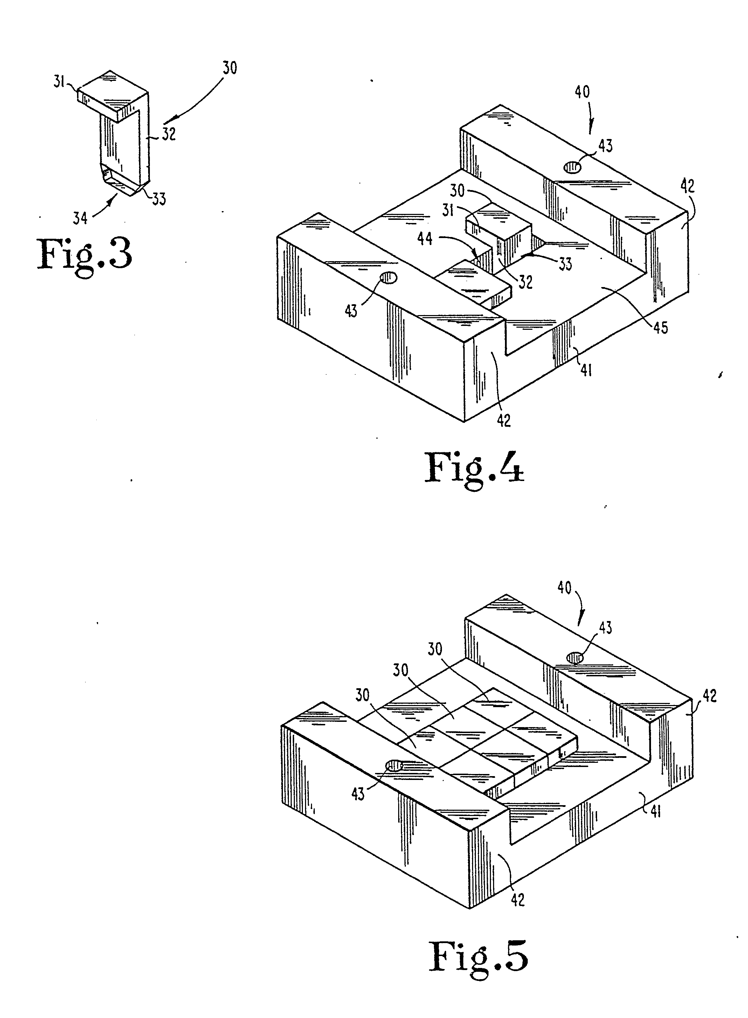

Referring now to FIG. 3, shown is an applicator tip 30 such as that available from Helena Laboratories under the name Micro-Tip (Catalogue Number 4087). The applicator tip 30 has a short segment 31 and a long segment 32 and generally forms an L shape. A generally U-shaped metallic piece 33 depends downwardly from the end of the long segment 32, and the metallic piece 33 has a slotted base 34 which forms a generally rectangular boundary to which a sample may adhere by surface tension.

Referring now to FIG. 4, shown is a generally U-shaped sample delivery piece 40 of applicants' preferred multi-tipped applicator device. The sample delivery piece 40 has a middle segment 41 having upwardly extending lip elements 42 at each end. Each of the lip elements 42 has a hole 43 extending completely through it, with the holes 43 being of a size such that guide bars (shown as 27 in FIG. 3) slide easily therethrough. The middle segment 41 has a rectangularly-shaped opening 44 through which the long segments 32 of applicator tips 30 are extended. The short segments 31 of the applicator tips 30 are not extended through the opening 44 but rather extend onto and are supported by the upper surface 45 of the middle segment 41. In this manner, the applicator tips 30 are supported by the sample delivery piece 40 with the long segments 31 of the applicator tips extending through the sample delivery piece 40 and beyond its bottom surface. Additionally, with this manner of support, each applicator tip 30 is independently vertically displaceable, which facilitates complete and uniform sample delivery to a slide even when the sample delivery piece 40 is slightly tilted (i.e. horizontally misaligned) with respect to the base piece 20.

In a more preferred embodiment, the rectangularly-shaped opening 44 in the sample delivery piece 40 is of a size and shape such that six of the applicator tips 40 may be extended therethrough and supported by the sample delivery piece 40. Referring to FIG. 5, this type of arrangment is illustrated, with two rows of three applicator tips 30 being supported by the sample delivery piece 40.

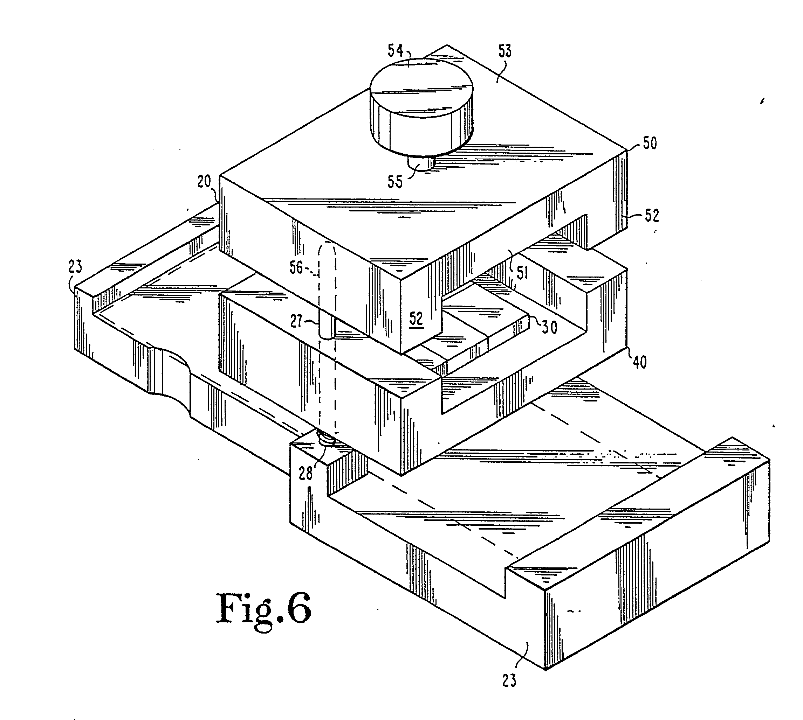

Referring now to FIG. 6, shown is a perspective view of several pieces of the applicants' preferred multi-tipped applicator device. A generally U-shaped top piece 50 is included and has a substantially planar segment 51 with leg elements 52 depending downwardly therefrom. Attached to the upper surface 53 of the top piece 50 is a cap 54. This attachment may be accomplished by a stem 55, which may either be threaded into the cap 54 and/or top piece 50 or glued thereto.

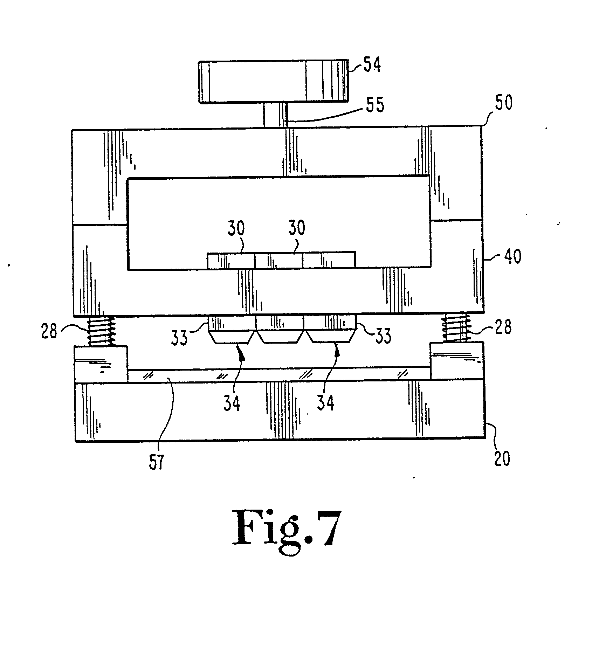

Each leg element 52 has a hole 56 extending partially therethrough into which a guide bar 27 may be comfortably slipped. The holes 43 in the sample delivery piece 40, and the holes 56 in the top piece 50 are correspondingly located such that the guide bar 27, which is snugly fitted in the holes 24 of the base piece 20, slips through them and aligns the two pieces over the base piece 20. The springs 28 slipped over the guide bars 27 resiliently bias the sample delivery piece 40 and the top piece 50 away from the base piece 20. In this manner, when a slide (contours indicated by dotted lines) is supported on the base piece 20, the applicator tips 30 are resiliently supported above the slide. Referring to FIG. 7, which is a right end view of FIG. 6 with the upwardly extending lip elements 23 of the base piece 20 having been broken away, a more clear view of the applicator tips 30 resiliently supported above a slide 57 is presented.

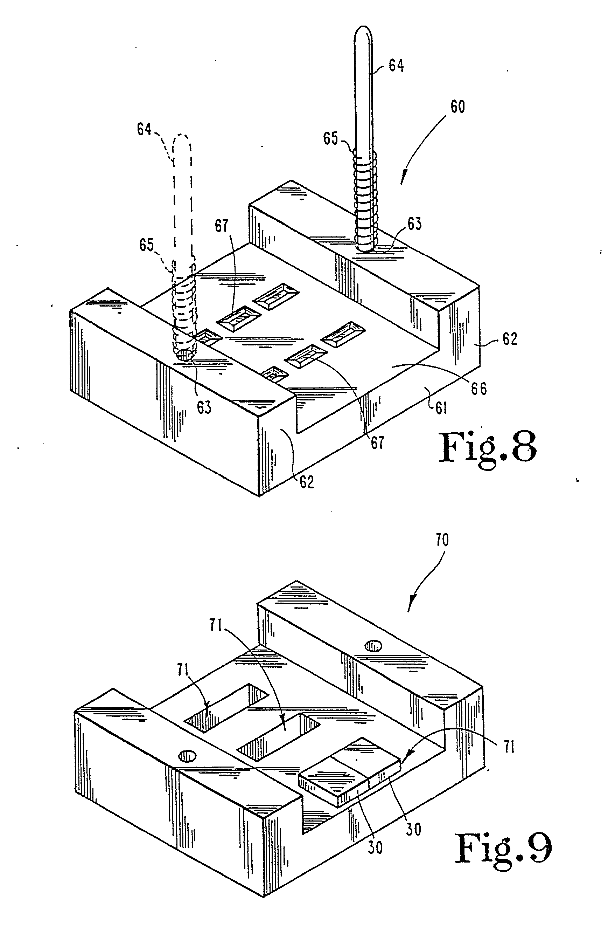

Referring now to FIG. 8, shown is a generally U-shaped charging station 60 which may be used to charge applicator tips with sample. The charging station 60 has a middle portion 61 and two upwardly extending leg elements 62. Each leg element 62 has a hole 63 extending partially therethrough into which a guide bar 64 may be snuggly fitted. A soft spring 65 is slipped over each guide bar 64. The inner surface 66 of the charging piece 60 has six separate wells 67 into which sample may be placed, with each well being capable of holding enough sample to charge an applicator tip several times.

Except for the guide bars 27 and 64, and the springs 28 and 65, all pieces of the applicants' preferred multi-tipped applicator device may be manufactured of plastic or the like, and the applicants have preferred to date to manufacture them out of plexiglass. To date, the guide bars and the springs have preferably been made of metal such as steel.

### Operation of the Preferred Multi-Tipped Applicator Device

Referring now to FIG.'s 2-8, the operation of the applicants' preferred multi-tipped applicator device will be explained. Desired samples are placed into the six wells 67 of the charging station 60. Thereafter, the sample delivery piece 40 supporting six applicator tips 30, as in FIG. 4, is slipped over the guide bars 64 of the charging station 60 and resiliently supported above the same by the springs 65. The six wells 67 of the charging station 60 are located such that they are aligned with and correspond to the slotted bases 34 of the applicator tips 30, with each well 67 corresponding to a different slotted base 34. The top piece 50 is thereafter slipped over the guide bars 64 of the charging station 60 and lowered until the legs 52 of the top piece 50 contact and are supported by the upwardly extending lip elements 42 of the sample delivery piece 40. Downard force is then applied to the cap 54 of the top piece 50 (as by tapping) to overcome the upward bias of the springs 65 and cause the slotted bases 34 of the applicator tips 30 to contact the sample in the wells 67 of the charging station 60. The downward force is then removed to allow the upward bias of the springs 65 to cause the slotted bases 34 of the applicator tips 30 to be removed from the wells 67. In this fashion, the applicator tips are charged as an amount of the contacted sample adheres to the slotted bases 34 of the applicator tips 30 by surface tension.

The top piece 50 and the sample delivery piece 40 (now with applicator tips charged) are thereafter slipped off of the guide bars 67 of the charging station 60, and subsequently slipped over the guide bars 27 of the base piece 20 onto which has previously been placed a glass slide. The resulting configuration is illustrated in FIG.'s 6 and 7. Downward force is then applied to the cap 54 of the top piece 50 (as by tapping) to overcome the upward bias of the springs 28 of the base piece 20. As a result, the slotted bases 34 of the applicator tips 30 contact the glass slide and the surface tension of the samples and the slotted bases 34 is thereby broken. Each applicator tip 30 thus deposits a generally rectangularly shaped uniform sample onto the glass slide.

The sample delivery piece 40 and the top piece 50 are thereafter slipped off of the guide bars 27 of the base piece 20, and the glass slide, now with deposited samples, may be removed from the base piece and further processed, if desired.

Referring now to FIG. 9, in another aspect of the applicants' multi-tipped applicator invention, a sample delivery piece 70 may be constructed and used as part of the applicants' multi-tipped applicator device. The sample delivery piece 70 may be identical to the above-discussed sample delivery piece (40 in FIG.'s 4-8) except instead of having a single rectangular opening capable of supporting two rows of three applicator tips (six total), the sample delivery piece 70 may have three smaller rectangular openings 71 each capable of supporting two applicator tips 30. When this sample delivery piece 70 is used as part of the applicants' multi-tipped applicator device (instead of the previously-discussed sample delivery piece 40), the resulting placement of biomaterial onto a slide is in an arrangement such as that illustrated in FIG. 1, i.e. three rows of two abreast. Thus, the multi-tipped applicator device may be designed so that the applicator tips correspond to and are in registry with pre-marked and/or numbered positions of a glass slide, providing a multi-tipped applicator device for the quick and efficient preparation of assay devices useful as controls or standards from pre-marked slides.

## Multi-Tipped Applicator Method

Referring now to FIG. s 1-9, another preferred aspect of the applicants' invention involves a method of preparing an assay device useful as a control or standard for analyzing a biomaterial. This aspect of the invention includes the steps of providing a glass slide 12, and then using a multi-tipped applicator device, such as the applicants' above-discussed preferred multi-tipped applicator device, to place biomaterial 16 into several separate identifiable areas of the glass slide 12, with each tip 30 of the applicator device placing one type of biomaterial 16 in a known amount into one of the several areas, differing from the type or amount of the biomaterial in each of the other several areas.

In one mode of practicing this aspect of the invention, the glass slide 12 may be prepared having several separate identifiable areas having biomaterial 16 of the same type, but in differing concentrations, and which also includes a separate identifiable area which has no concentration of the type of biomaterial in the other several areas, as a negative control. For example, into areas of the glass slide 12 marked 1-5 could be placed 5 serial dilutions of IgG, with each area receiving a different dilution. A solution of Bovine Serum Albumin could be placed into the area marked 0 to form a negative control.

This may be accomplished by placing serial dilutions of the desired biomaterial 16 into 5 of the wells 67 of the charging station 60, with each well 67 receiving a different dilution. Into the sixth well 67 may be placed a solution of Bovine serum albumin. Thereafter, the multi-tipped applicator device may be used as described above to result in the placement of the desired biomaterial 16 of differing concentrations to the 5 separate identifiable areas of the glass slide marked 1-5, with the sixth separate identifiable area marked 0 receiving a concentration of Bovine Serum albumin, to be used as a negative control.

It is contemplated that other means of delivering the biomaterial to the tips of the multi-tipped applicator device could be used. Additionally, any type of biomaterial which may have been discussed with regard to the above aspects of the invention could also be used in this aspect of the invention, with more preferred biomaterials being antigens, antibodies, enzymes, and hormone receptors. Also, suitable fixatives and either non-coated or gelatin-coated glass slides could be used.

Any multi-tipped applicator device having at least 3 tips in any arrangement suitable for application of biomaterial to a glass slide could be used in this aspect of the invention, although the most preferred applicator has at least 6 tips, most preferably arranged in a rectangular array.

In another mode of practicing this aspect of the applicants' invention, instead of having differing concentrations of one type of biomaterial 16 in the wells 67 of the charing station 60, the wells 67 may contain biomaterial 16 consisting of cells or organelles, with differing morphology or reactivity in each of the wells 67. In this manner, when the multi-tipped applicator device is used as described above, an assay device may be formed which has biomaterial of differing cell or organelle reactivity or morphology in each of the several separate identifiable areas. For example, the glass slide 12 could be prepared having PAP cells 0-4 in the areas marked 0-4, with each area receiving a different type (0-4) of PAP cell.

In a more preferred mode of this aspect of the applicants' invention, the glass slide 12 has been treated as follows before the biomaterial 16 has been applied with the multi-tipped applicator device. First the glass slide has been etched by treating it with acid. The most preferred acid to date has been acetic acid, although any other acid capable of etching the surface of the glass slide could be used. Second, the glass slide has been rinsed with distilled water. Third, the slide has been rinsed with alcohol, and although any suitable alcohol could be used, the applicants' preferred alcohol to date has been ethanol. Fourth, the slide has been dried, preferably at an elevated temperature and most preferably at about 37° C. Fifth, the glass slide, or at least a portion of a surface thereof, has been coated with a layer of gelatin, and the layer has been dried preferably at an elevated temperature, most preferably at about 37° C. To date, the applicants' preferred method of coating the glass slides has been immersing them in a .5% solution of gelatin. Finally, after the multi-tipped applicator device has been used to apply the biomaterial to the slide, the biomaterial is treated with a suitable fixative.

## Method for Preparing a Gelatin Coated Assay Device

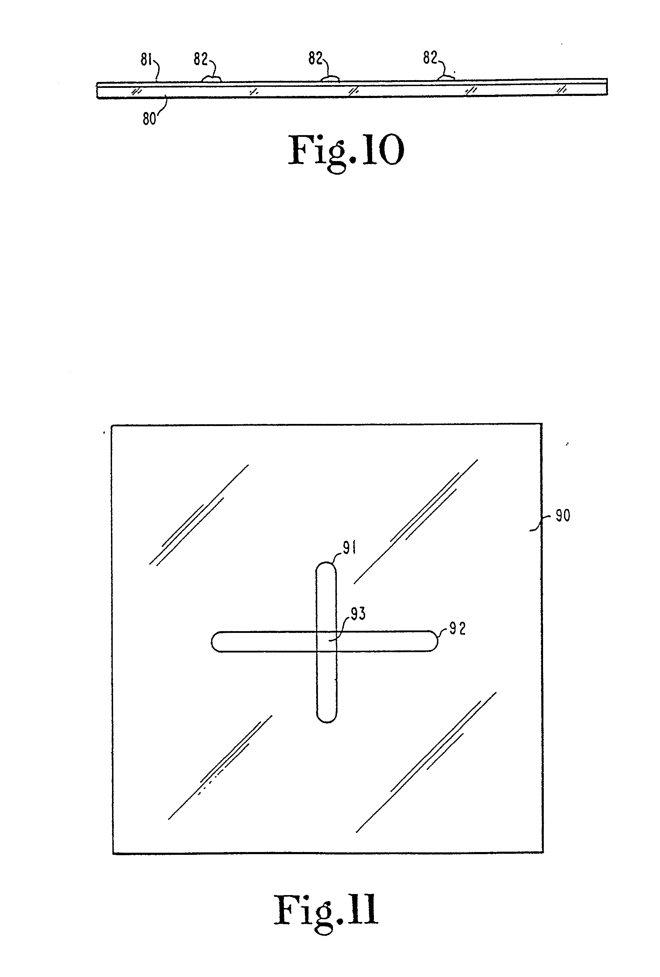

Still another aspect of the invention involves a method of preparing an assay device useful as a control or standard for analyzing a biomaterial. Referring now to FIG. 10, this aspect of the applicants' invention is highlighted by the steps of selecting a glass slide 80, coating at least a portion of one of its surfaces with a layer of gelatin 81, and placing differing types or amounts of biomaterial 82 in known amount into each one of several separate identifiable areas of the glass slide on the surface coated with gelatin so that each of the several areas has only one type or amount of the biomaterial.

Again, the biomaterial 82 may be any biomaterial which may have been discussed in the above aspects of the invention, including but not limited to cells, such as cells for PAP testing, parts of cells, viruses, antigens, antibodies, enzymes, hormone receptors, and nucleic acids such as RNA and DNA. The gelatin coating step is preferably accomplished by dipping the slide in a .5% solution of gelatin, although other means such as spraying one or more of the surfaces of the slide with a gelatin solution are contemplated. In a more preferred form, the glass slide is etched with acid prior to the gelatin coating step, with the most preferred acid for this step being acetic acid. Additionally, after the biomaterial has been applied and dried, the slide is preferably treated with a suitable fixative.

## Cross Pattern Method

Referring now to FIG. 11, yet another aspect of the present invention involves a novel method for preparing an assay device useful as a control or standard which is highlighted by the steps of providing a glass slide 90, and placing a first sample of biomaterial in a generally elongated area 91 of the glass slide 90, and placing a second sample of biomaterial in a second elongated area 92 of the glass slide 90 so that the two sample areas 91 and 92 intersect, as at 93, and thus generally form a cross pattern. Only one type of biomaterial in a known amount is placed in each of the sample areas 91 and 92, differing from the type or amount in the other area. In such a manner, three sample area types are formed, one consisting of said first sample, one consisting of said second sample, and one consisting of a combination of said first and second samples. Any biomaterial could be used in this aspect of the invention, including but not limited to cells, such as cells for PAP testing, parts of cells, viruses, antigens, antibodies, enzymes, hormone receptors, and nucleic acids such as RNA and DNA. To date, the applicants' have preferably used the above-described Micro-Tips or Macro-Tips available from Helena Laboratories to place the biomaterial on the slide. Additionally, the slide may be pre-treated and/or coated with a layer of gelatin, and the biomaterials may be treated with a suitable fixative after being placed on the slide, as described in the above description and in the Examples below.

In one mode of practicing this preferred aspect of the invention, biomaterial of the same type is placed in differing concentrations in each of the sample areas 91 and 92. For example, a first serial dilution of IgG may be placed in sample area 91, and a second serial dilution may be placed in sample area 92. An assay device thus prepared would have three identifiable areas of differing concentrations of IgG, and would be useful as a control or standard for quantitative and/or qualitative testing for the presence of the antigen IgG in an unknown, or for checking IgG assay procedures, reagents, or equipment for proper function.

In another mode of practicing this aspect of the invention, biomaterials of differing types are placed in sample areas 91 and 92, with each area receiving only one biomaterial type. For example, single stranded DNA could be placed in sample area 91, and double stranded DNA could be placed in sample area 92. The device thus prepared has three identifiable sample area types, one consisting of single stranded DNA, one consisting of double stranded DNA, and one consisting of a mixture of single stranded and double stranded DNA. Such an arrangement is useful in qualitative determination of unknowns, and in checking assay procedures and equipment for proper function.

For the purposes of further promoting a better understanding of the assay devices and methods for their preparation of the present invention, reference will now be made in the Examples below to specific instances of their preparation and use. These examples are exemplary only, and no limitation of the scope or breadth of the applicants' invention is intended thereby.

## EXAMPLE 1

24 glass slides were soaked for 30 minutes in a 5% solution of acetic acid. The slides were then soaked in distilled water for 5 minutes, and thereafter soaked in ethanol for about 20 minutes. The slides were then dried in an oven for about 30 minutes at 35° C, and subsequently dipped into a 0.5% solution of gelatin. The gelatin-coated slides were dried in an oven overnight at about 35° C, and the next day, using a Helena Micro-Tip, five of the slides were spotted with IgGK, each receiving the biomaterial in 9 separate identifiable areas, and subsequently dried in an oven for periods of time ranging from 1 to 5 hours. Successful staining of slides thus prepared was obtained using Amido Black.

## EXAMPLE 2

24 gelatin-coated slides were prepared using the method for preparing the same outlined in Example 1 above. IgG, IgM, and gamma globulin were each used to spot 8 of the slides, with each slide being spotted in 9 separate identifiable areas. The biomaterial was then fixed for one-half hour with formalin vapor at 35° to 37° C. The slides were then dried for two hours in an oven at 35° to 37° C, whereafter they were successfully stained using Tissue-Tek Avidin-Biotin (AB) System, Miles Scientific.

## EXAMPLE 3

Six of the gelatin-coated slides of Example 1 were used in this Example. Five dilutions of single stranded DNA (Sigma, lot # 75f-66542) were prepared as follows. 5 milligrams of the DNA were mixed with 1 milliliter of distilled water, forming the first dilution. A second dilution was prepared by adding to 100μl of the first dilution 200μl of PBS. To 100μl of this second dilution was added 200 μl of PBS to form the third dilution. Fourth and fifth dilutions were similarly prepared. Each of 5 separate identifiable areas of each of the slides were then spotted with the 5 dilutions, with each area on a slide receiving a different dilution. A sixth separate identifiable area on each slide was spotted with 1% Bovine Serum Albumin (Sigma lot #97f04), as a negative control. The slides were then air dried, fixed with acetic alcohol (1:3) for 45 minutes, and air dried again. The spotted areas were then coated with a layer of melted paraffin, and the slides stained using the Feulgen procedure with "Blue Feulgen" as the Schiff reagent (see e.g. Staining Procedures, Fourth Ed., Clark, George, Williams and Wilkins, Baltimore & London 1981 pp. 201-292). The first through third areas showed positive but decreasing levels of staining, and the fourth through sixth areas showed no visible staining, giving indication of the range of concentration of single stranded DNA for which this staining procedure may be effectively used, providing a standard or control to which an unknown may be compared, and demonstrating the use of a negative control (sixth area).

## EXAMPLE 4

Example 3 is repeated except the glass slides used are not pre-treated and not coated with a layer of gelatin.

## EXAMPLES 5-6

Examples 3 and 4 are repeated except glass slides marked with indicia which divide the surface of the slide into 6 separate identifiable areas are used, and the samples are placed into the separate identifiable areas.

## EXAMPLES 7-8

Examples 3 and 4 are repeated except the applicants' preferred glass slides marked numerically (0-5) and with indicia are used, with the 5 DNA dilutions being placed at numerically marked positions 1-5 and the BSA being placed at numerically marked position 0.

## EXAMPLE 9

Four of the gelatin-coated slides of Example 1 were used in this Example. To $100\mu l$ of double stranded DNA was added $100\mu l$ of 1 N NaOH, whereafter this dilution was used to spot first through third areas of each of the slides. Fourth through sixth areas of each slide were each spotted with the first single stranded DNA dilution described Example 3 above. The slides were then stained using Ponceau. The results showed staining at all 6 areas, however the fourth through sixth areas with single stranded DNA stained differently than the first through third areas with double stranded DNA. The single stranded DNA areas stained with a more intense color, but with hazy, muted edges. The double stranded DNA areas stained lighter but showed more definition, thus demonstrating how the differing types of DNA used stained using this procedure and providing a standard or control to which an unknown may be compared.

## EXAMPLE 10

Example 5 is repeated except the glass slides used are not coated with a layer of gelatin.

## EXAMPLES 11-12

Examples 9 and 10 are repeated except the glass slides used are marked with indicia which divide the slides into six separate identifiable areas, and the samples are placed into such areas.

## EXAMPLES 13-14

Examples 9 and 10 are repeated except the applicants' preferred glass slides marked numerically (0-5) and with indicia available from Erie Scientific Company are used, with the 3 dilutions of double stranded DNA being placed at numerically marked positions 0-2, and the 3 dilutions of single stranded DNA being placed at numerically marked positions 3-5.

## EXAMPLE 15

A gelatin-coated slide was prepared using the method for preparing the same outlined in Example 1. Nine serial dilutions of IgG were prepared as follows. $100\mu l$ of IgG, 1 g%, (Sigma lot #107F8822) served as the first dilution. To this was added $200\mu l$ of $Na_2PO_4$ buffer at pH 7.6 to form the second dilution. To $100\mu l$ of this second dilution was added $200\mu l$ of $Na_2PO_4$ buffer at pH 7.6 to form the third dilution. This procedure of adding to $100\mu l$ of each successive dilution $200\mu l$ of $Na_2PO_4$ buffer at PH 7.6 was repeated until 9 dilutions had been prepared. Each of 9 separate identifiable areas of the slide was then spotted with the 9 dilutions, each receiving a different dilution. The slide was then air dried and fixed in formaldehyde vapor in a 37° C oven for 30 minutes, and subsequently dried in a 37° C oven for one and one-half hours. The slide was then stained using kit #AGO47MA using poly Ab IgG (#R050027) as the primary antibody. The results showed staining at the first through sixth areas which contained the first six dilutions, and no visible staining at the seventh through ninth areas which contained the seventh through ninth dilutions, giving indication of the range of concentration of IgG for which this staining procedure may be effectively used, and providing a standard or control to which an unknown may be compared.

## EXAMPLE 16

Example 15 is repeated except the glass slides used are not coated with a layer of gelatin.

## EXAMPLES 17-18

Examples 15 and 16 are repeated except the glass slides used are marked with indicia which divide the surface of the glass slide into 9 separate identifiable areas, and the IgG samples are placed in such areas.

## EXAMPLES 19-20

Examples 15 and 16 are repeated except the applicants' preferred glass slides marked numerically (0-5) and with indicia available from Erie Scientific Company are used, with only the first 6 IgG dilutions being used and being placed at numerically marked positions 0-5.

EXAMPLE 21

A gelatin-coated slide is prepared using the method for preparing the same outlined in Example 1. Into five wells of the charging station of applicants preferred multi-tipped applicator device are placed five dilutions of single stranded DNA, and into the sixth well is placed a 1% Bovine Serum Album solution (control). The applicants' preferred multi-tipped applicator device is then used as described above to place the samples into several separate identifiable areas of the gelatin coated slide, noting throughout the procedure which applicator tips contact which solutions, and where each tip contacts the glass slide. The slide is then air dried and the biomaterials fixed with acetic alcohol (1:3) for 45 minutes, and air dried again. The slides are then stained using the Feulgen procedure with "Blue Feulgen" as the Schiff reagent, as in Example 3.

EXAMPLE 22

Example 21 is repeated except the slide used is not coated with a layer of gelatin.

EXAMPLES 23-24

Examples 3-14 are repeated except the applicants' multi-tipped applicator device is used to apply the samples to the slides.

EXAMPLES 35-40

Examples 15-20 are repeated except estrogen dilutions are used as the samples, formalin is used as the fixative, and appropriate known techniques for staining estrogen samples are used.

EXAMPLES 41-46

Examples 15-20 are repeated except cholinesterase dilutions are used as the samples, graded alcohol is used as the fixative, and appropriate known techniques for staining cholinesterase samples are used, such as those described by Jennings and LaFlore, "Staining Procedures", Fourth Ed. by George Clark, p. 277.

EXAMPLES 47-52

Examples 15-20 are repeated except anti IgG dilutions are used as the samples, formalin is used as the fixative, and appropriate known techniques for staining anti IgG samples are used.

EXAMPLE 53

A gelatin-coated glass slide is prepared as in Example 1. First through fourth separate identifiable areas are placed PAP cells 0-4, with each area receiving a different cell type. The cells are then fixed using a proper fixative, such as graded alcohol, and stained using a gram stain.

EXAMPLE 54

Example 53 is repeated except the glass slide used is not coated with a layer of gelatin.

EXAMPLES 55-56

Examples 53 and 54 are repeated except the glass slides used are marked with indicia which divide the surfaces of the slides into separate identifiable areas, and the PAP cells are placed into such areas.

EXAMPLES 56-57

Examples 53 and 54 are repeated except the applicants' preferred glass slides marked numerically and with indicia available from Erie Scientific Company are use, with the PAP cells 0-4 being correspondingly placed into the areas marked 0-4 of the slide.

## EXAMPLES 58-62

Examples 54-57 are repeated except applicants' preferred multi-tipped applicator device is used to deliver the samples to the slides.

## EXAMPLE 63

A gelatin-coated glass slide is prepared as in Example 1. A Helena Micro-Tip is used to place a sample of the first IgG dilution of Example 15 into a first generally elongated rectangular area of the slide. Thereafter, a Helena Micro-Tip is used to deliver a sample of the second IgG dilution of Example 15 to a generally elongated area of the glass slide wherein the first and second areas intersect to form an area with a combination of the first and second IgG samples. The slide is thereafter stained as in Example 15.

## EXAMPLE 64

Example 63 is repeated except the first single stranded DNA dilution and the first double stranded DNA dilution of Example 9 are used instead of the IgG dilutions, and the slide is stained using Ponceau as in Example 9.

## Claims

1. An assay device useful as a control or standard for analyzing a biomaterial comprising:
a glass slide having a surface
indicia which divide said surface into several separate identifiable areas; and
biomaterial placed into said several areas with only one type in a known amount in each of said several areas, differing from the type or amount in each other of said several areas, whereby procedures and equipment can be checked for proper function.

2. The device of claim 1 in which the several separate identifiable areas have biomaterial of the same type, but in differing concentrations of a substance to be assayed, and which also includes a separate identifiable area which has no concentration of the biomaterial, as a negative control.

3. The device of claim 1 in which the biomaterial consists of cells or parts therof, viruses or parts thereof, or organelles, and cells or parts therof, viruses or parts thereof, or organelles with differing morphology are placed in in each of said several separate identifiable areas, but uniform within each area.

4. The device of claim 1 in which the biomaterial consists of cells or parts therof, viruses or parts thereof, or organelles, and cells or parts therof, viruses or parts thereof, or organelles with differing reactivity are placed in each of said several separate identifiable areas, but uniform within each area.

5. The device of claim 1 in which the slide has a coating of gelatin and the biomaterial is adjacent the coating of gelatin.

6. An assay device useful as a control or standard for analyzing an antibody, antigen, enzyme, or hormone receptor comprising:
a glass slide having a substantially planar surface having several separate identifiable areas; and
an analyte selected from the group consisting of antibody, antigen, enzyme, and hormone receptor placed into said several areas with only one uniform concentration of said analyte in each of said several areas, and each of said several areas having differing concentrations of said analyte from each of the other of said several areas, whereby procedures and equipment can be checked for proper function in assaying said analyte.

7. The device of claim 6 in which said surface is visibly divided into said several areas by indicia.

8. A multi-tipped applicator device for placing uniform samples of biomaterial into several separate identifiable areas of a glass slide comprising:
a charging station having several separate wells for holding sample, said charging station being operably associated with a first vertical guide means;
a base piece for supporting a glass slide, said base piece being operably associated with a second vertical guide means;
a sample delivery piece for supporting several applicator tips wherein each tip is independently vertically displaceable, said sample delivery piece being displaceable along said first vertical guide means toward and away from said charging station to cause said applicator tips supported by said sample delivery piece to temporarily contact said sample in said wells of said charging station thus charging said applicator tips with sample;
said sample delivery piece also being vertically displaceable along said second vertical guide means toward and away from said glass slide supported by said base piece to cause said applicator tips supported by said sample delivery piece to temporarily contact said glass slide and thus deposit said sample on said glass slide; and
a top piece displaceable along said first and second vertical guide means and designed to facilitate displacement of said sample delivery piece along said first and second vertical guide means.

9. The device of claim 8 wherein said applicator tips comprise a metallic piece, said metallic piece having a slotted base which forms a generally rectangular boundary to which the sample adheres by surface tension.

10. The device of claim 8 wherein said applicator tips are positioned in an array which is in registry with several numerically marked separate identifiable areas of a glass slide to be charged with sample by said multi-tipped applicator device.

11. A method of preparing an assay device useful as a control or standard for analyzing a biomaterial comprising the steps of:

(a) providing a glass slide having a surface;

(b) providing biomaterial to the tips of a multi-tipped applicator; and

(c) using said multi-tipped applicator to place said biomaterial into several separate identifiable areas of said surface, each tip of said applicator placing one type of said biomaterial in a known amount into one of said several areas, differing from the type or amount of said biomaterial in another of said several areas.

12. The method of claim 11 in which the surface of the glass slide is divided by indicia which correspond to each of the several separate areas.

13. A method of preparing an assay device useful as a control or standard for analyzing a biomaterial comprising the steps of:

(a) providing a glass slide;

(b) coating at least one surface of said glass slide with gelatin; and

(c) placing differing types or amounts of biomaterial in known amount into each one of several separate identifiable areas of said glass slide on the surface coated with gelatin so that each of said several areas has only one type or amount of the biomaterial.

14. The method of claim 13 in which said coating is preceded by the step of etching the surface of said slide.

15. A method of preparing an assay device useful as a control or standard for analyzing a biomaterial comprising the steps of:

(a) providing a glass slide;

(b) placing differing types or amounts of biomaterial in known amount into first and second areas of said glass slide, each of said first and second areas receiving only one type or amount of biomaterial, and wherein said areas intersect forming a third area which contains a combination of said biomaterial in said first and second areas.

Fig.1

Fig.2

Fig.3

Fig.4

Fig.5

Fig.6

Fig.7

Fig.8

Fig.9

Fig.10

Fig.11